Europäisches Patentamt

(19) European Patent Office

Office européen des brevets

(11) Numéro de publication: **0 172 292**
**A1**

(12) # DEMANDE DE BREVET EUROPEEN

(21) Numéro de dépôt: **84401707.9**

(22) Date de dépôt: **22.08.84**

(51) Int. Cl.⁴: **C 02 F 11/02**
**C 02 F 11/16, C 05 F 13/00**
**C 12 M 1/00**

(43) Date de publication de la demande:
**26.02.86 Bulletin 86/9**

(84) Etats contractants désignés:
**AT BE CH DE GB IT LI NL SE**

(71) Demandeur: **MULTIBIO, Société Anonyme dite**
**45, rue de la Chaussée d'Antin**
**F-75009 Paris(FR)**

(72) Inventeur: **Legesgue, Yves**
**Le Relais - 34 Grande Rue**
**Commeny - 95450 Vigny(FR)**

(72) Inventeur: **Zeana, Alexandru**
**10-12, Allée de la Toison d'Or**
**F-94000 Creteil(FR)**

(74) Mandataire: **Cuer, André et al,**
**CABINET CUER 30, rue de Léningrad**
**F-75008 Paris(FR)**

(54) **Procédé et installation pour la production simultanée de compost et de biogaz à partir de déchets organiques.**

(57) L'invention concerne un procédé de traitement simultané de déchets organiques solides ou semi-solides et de déchets organiques liquides en vue de la production simultanée de compost et de biogaz.

Selon l'invention les déchets organiques liquides sont soumis à une séparation liquide-solide (6), la phase liquide (15) provenant de cette séparation est soumise à une fermentation anaérobie dans au moins un digesteur fermé (16), la phase solide (7) provenant de ladite séparation liquide-solide est mélangée auxdits déchets organiques solides ou semi-solides (3), le mélange résultant (12) est soumis à une fermentation aérobie (13) à la périphérie dudit digesteur (16) et en contact avec celui-ci et l'on évacue dudit digesteur respectivement des boues (4), du liquide clarifié (28) et du gaz (25), tandis que l'on récupère (29) à la périphérie dudit digesteur du compost provenant de la fermentation aérobie desdits déchets solides ou semi-solides.

./...

EP 0 172 292 A1

FIG.1

0172292

## Procédé et installation pour la production simultanée de compost et de biogaz à partir de déchets organiques

La présente invention a trait au domaine de la valorisation de déchets liquides et/ou solides de diverses sources et nature. Elle concerne plus spécialement le traitement, à la fois en atmosphère extérieure (phase aérobie) et en milieu clos (phase anaérobie), de déchets et matières polluées organiques, respectivement solides ou semi-solides et liquides, en vue de la production directe et simultanée de compost, de biogaz et deliquide épuré.

Le volume croissant des déchets et résidus de tous types constitue un problème de plus en plus préoccupant pour les industriels et les municipalités, compte tenu notamment du fait que ces déchets présentent généralement de grands dangers pour l'environnement et pour les êtres vivants.

Bien souvent, on adopte la solution, pour les résidus liquides, de solidification de ces déchets par un traitement physico-chimique donnant lieu à une pétrification, les masses dures obtenues restant sur le site de traitement ou pouvant être utilisées pour la réalisation de structures de fondation ou divers revêtements de sols.

Selon un autre objectif, on peut chercher à valoriser une partie au moins de ces déchets, par exemple en visant la production d'énergie calorifique ou, mieux encore, lorsqu'il s'agit de résidus organiques, plus ou moins riches en carbone et autres substances non minérales, en mettant en oeuvre les techniques connues de fermentation en milieu aérobie et/ou anaérobie en vue d'obtenir soit des composts, engrais et divers amendements pour les sols, soit une production de gaz dit "biogaz" riche en méthane et utilisable comme combustible pour l'obtention d'énergie thermique ou électrique ou encore pour la fabrication de produits chimiques.

L'invention se situe dans ce domaine de valorisation des déchets organiques et a essentiellement pour but, à la fois, de transformer des déchets organiques solides en matériaux utiles notamment pour l'agriculture et d'assurer le traitement de déchets organiques liquides en vue de les dépolluer et d'obtenir d'une part du biogaz et d'autre part des fluides épurés directement utilisables pour l'industrie ou la consommation.

Parmi les déchets ou sous-produits solides utilisables comme matières premières dans l'invention on peut citer en particulier: des fractions de tri d'ordures ménagères, des pailles, des sciures et écorces de bois et matériaux ligneux, des pulpes de raisin, drêches, de la bagasse, des tourbes, des herbacées, etc.

Quant aux résidus liquides, ils peuvent concerner en particulier tous les rejets concentrés provenant notamment des industries agricoles et agro-alimentaires telles que par exemple: sucreries, distilleries vinicoles, brasseries et industries de la fermentation, abattoirs et conserveries de viande, industries laitières, industries de la féculerie, porcheries, etc. ainsi, bien entendu, que toutes les boues primaires et des boues biologiques en excès récupérées dans les stations d'épuration des eaux urbaines.

Dans l'exposé qui suit, on utilisera également le terme de déchets ou résidus semi-solides pour désigner des matériaux normalement solides, mais qui ont une teneur en eau généralement comprise entre 30 % et 90 %, par exemple de l'ordre de 45 à 80 % environ.

Il est déjà bien connu d'assurer la fermentation en tas, donc en milieu aérobie, de divers résidus agricoles solides pour la production de compost. Toutefois, il s'agit d'opérations longues qui se prêtent généralement très mal à l'industrialisation. Par ailleurs, on sait produire du biogaz par fermentation anaérobie de résidus semi-solides ou de boues dans des digesteurs; toutefois, ces appareils doivent être habituellement chauffés pour obtenir un rendement correct et la masse à traiter doit être agitée et brassée; en outre, la concentration des boues doit être faite dans des décanteurs, par exemple lamellaires, séparés du digesteur.

Le but essentiel de l'invention est de proposer un procédé associant les deux types de fermentation aérobie et anaérobie de façon telle que l'on puisse assurer le traitement simultané de déchets solides ou semi-solides et de déchets liquides afin d'obtenir, à la sortie d'une seule installation, la production: de matériaux solides riches en substances fertilisantes, de biogaz apte à servir de combustible ou autre et, enfin, de liquides épurés immédiatement réutilisables et admissibles par l'environnement.

Un autre but est d'utiliser les calories de la fermentation aérobie pour assurer, sans apport extérieur, l'équilibre thermique idéal de la digestion anaérobie des déchets ou résidus liquides.

Un autre but, encore, est de fournir un type de digesteur ne nécessitant aucun organe mécanique d'agitation et dans lequel le brassage des matières boueuses est effectué par la seule pression du gaz généré par la fermentation en milieu clos.

Enfin, d'autres buts apparaîtront encore dans la description qui suit, notamment la mise en oeuvre de micro-organismes et/ou enzymes, de préférence fixés sur certains supports, dans le but d'accélérer les processus de transformations biologiques et d'accroître les rendements de production dans les unités industrielles.

Pour résoudre les problèmes et atteindre les résultats susvisés, l'invention propose un procédé de traitement simultané de déchets organiques solides ou semi-solides et de déchets organiques liquides en vue de la production simultanée de compost et de biogaz, caractérisé en ce que les déchets organiques liquides sont soumis à une séparation liquide-solide, en ce que la phase liquide provenant de cette séparation est soumise à une fermentation anaérobie dans au moins un digesteur fermé, en ce que la phase solide provenant de ladite séparation liquide-solide est mélangée auxdits déchets organiques solides ou semi-solides, en ce que le mélange résultant est soumis à une fermentation aérobie à la périphérie dudit digesteur et en contact avec celui-ci, et en ce que l'on évacue dudit digesteur respectivement des boues, du liquide clarifié et du gaz, tandis que l'on récupère à la périphérie dudit digesteur du compost provenant de la fermentation aérobie desdits déchets solides ou semi-solides.

Les boues de digestion recueillies à la partie inférieure du fermenteur clos sont avantageusement recyclées à la phase de mélange et de broyage des déchets carbonés solides, par exemple à la partie supérieure du composteur comme on le verra plus loin, en vue d'assurer une humidification de ces déchets jusqu'à au moins 55 % et de subir également le traitement aérobie. En outre, la fraction sédimentaire solide des matières premières liquides à traiter (comme par exemple des eaux résiduaires brutes) est récupérée et additionnée à la masse des résidus à composter en atmosphère libre.

Le traitement aérobie favorise, comme on le sait, l'hydrolyse du substrat et produit, notamment par métabolisation des polysaccharides, une grande quantité d'énergie sous forme de calories qui, selon l'invention, sont utilisées pour chauffer les parois du digesteur. Ce dernier se trouve donc naturellement maintenu à une température variant de 35°C à 50°C environ selon l'état d'avancement du processus aérobie.

L'introduction des résidus-liquides dans le milieu anaérobie peut être faite de haut en bas, mais, plus avantageusement, de bas en haut dans le digesteur. En outre, dans la zone périphérique de compostage comme au sein du digesteur, il est souvent utile d'introduire un inoculum choisi par exemple dans le groupe des levures, bactéries, enzymes afin de favoriser les cinétiques des réactions de décomposition des substrats et d'engendrer la formation d'ilots de prolifération de microorganismes en phase aérobie, ainsi que d'accélérer la phase de méthanogénèse - généralement lente - dans le milieu anaérobie, par exemple par ensemencement de bactéries appropriées telles que méthanosarcina, également fixées sur support.

Selon un mode de réalisation préféré, l'incorporation de ces inoculum se fait en milieu fixé, les produits de fixation étant constitués avantageusement par de fines particules, de granulométrie 0,1 à 100 microns environ, de produits minéraux comme par exemple: des verres poreux, gels de silice, divers oxydes métalliques ou encore des argiles cuites (ou chamottes) qui conviennent particulièrement bien. Dans le digesteur, comme on le verra plus loin, on met avantageusement en oeuvre des plaques, en système lamellaire, qui servent à la fois de supports fixes pour l'immobilisation des enzymes (par exemple) et de moyens de récupération des boues résiduelles du liquide à épurer.

L'invention a également pour objet une installation pour le traitement simultané de déchets organiques solides ou semi-solides et de déchets organiques liquides en vue de la production simultanée de compost et de biogaz, caractérisée en ce qu'elle comprend: des moyens pour séparer les déchets organiques liquides en une phase solide et une phase liquide; au moins un digesteur fermé; des moyens pour introduire ladite phase liquide dans ledit digesteur; des moyens pour mélanger ladite phase solide auxdits déchets solides ou semi-solides; des moyens pour amener le mélange résultant à la périphérie dudit digesteur en contact avec celui-ci; et des moyens pour évacuer respectivement dudit digesteur, d'une part, le gaz qui s'y forme par fermentation anaérobie, d'autre part, les boues qui s'y déposent, enfin, la phase liquide clarifiée.

Avantageusement, le digesteur est pourvu de plaques verticales pour la fixation des bactéries et l'écoulement des boues au fond du radier, ces plaques pouvant, par exemple, être fixées sur une grille inférieure qui délimite le compartiment du radier.

Le stockage du biogaz produit par le fermenteur anaérobie peut être effectué de diverses façons. Par exemple, le fermenteur peut être muni sur sa partie supérieure d'un gazomètre ou cloche dont la pression de service est maintenue constante et contrôlée par manomètre. Selon une variante, l'installation peut inclure un gazomètre souple, par exemple en tissu nylon imprégné de néoprène et hypalon (ou autres matériaux équivalents), installé sous abri pour assurer sa protection.

De nombreuses autres caractéristiques apparaîtront dans la description qui suit, relative à des modes de réalisation non limitatifs et illustrés par les dessins annexés, qui représentent schématiquement:
Figure 1, une vue générale de principe d'une installation de mise en oeuvre du procédé de l'invention;

Figure 2, une vue en coupe, simplifiée, d'un digesteur selon l'invention muni ici d'une cloche à gaz;

Figures 3 et 4, des représentations de montage de plusieurs digesteurs (ici deux) respectivement en série et en parallèle dans une unité de production industrielle;

Figure 5, l'illustration schématique d'un ensemble modulaire composite: fermenteurs aérobie et digesteurs anaérobie sous forme de structure en "nid d'abeilles".

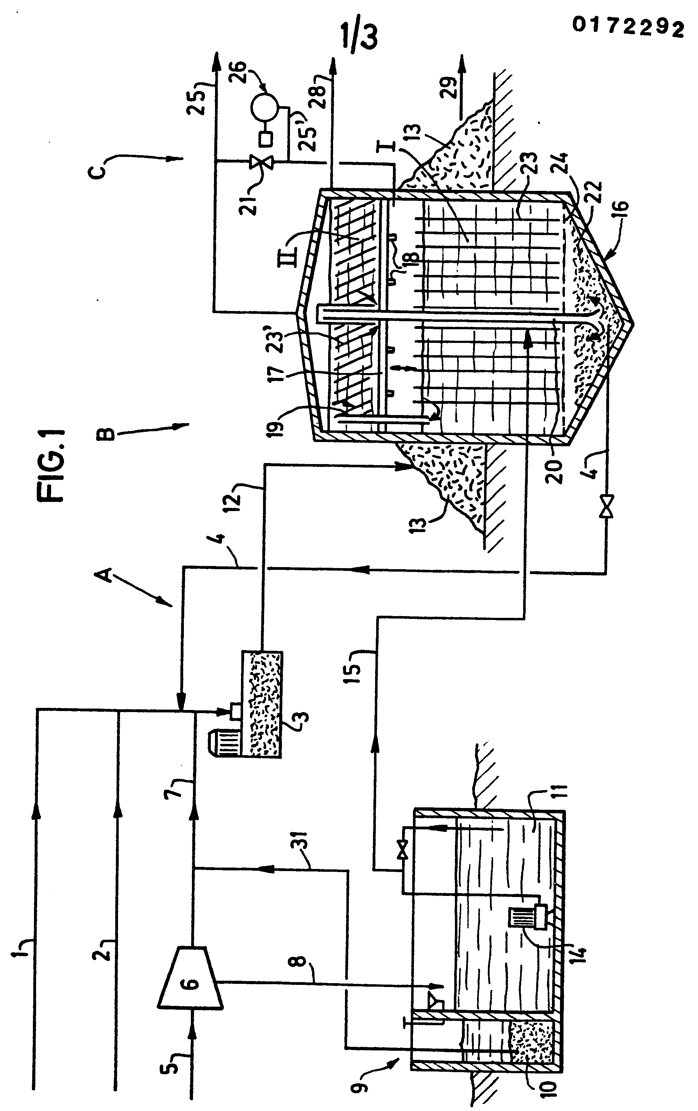

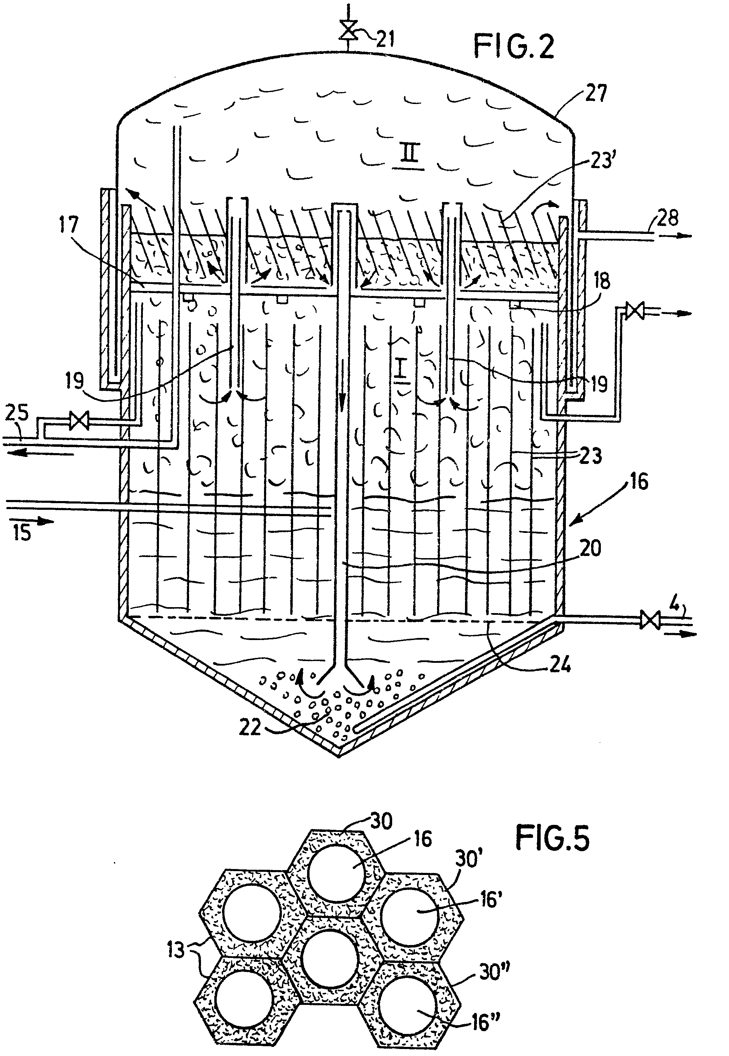

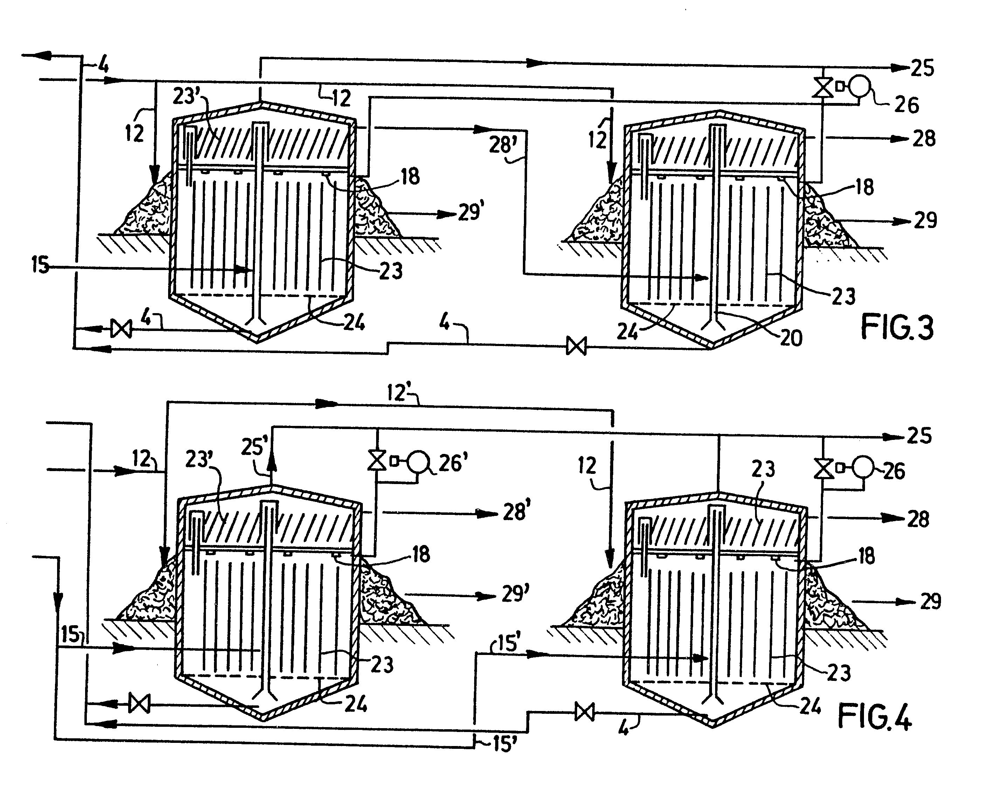

Telle que représentée sur les dessins, en particulier sur la figure 1, une installation de production simultanée de compost, biogaz et liquide épuré selon l'invention comprend essentiellement, de gauche à droite: une partie amont A relative aux traitements préalables et à l'amenée des matières premières (déchets solides, semi-solides et liquides); la partie centrale B proprement opérationnelle pour la mise en oeuvre du procédé mixte: aérobie-anaérobie; et la zone aval C de récupération et éventuellement traitement des produits finaux obtenus.

On se référera tout d'abord à la fraction amont A. Les déchets carbonés solides (bois, paille, écorces, sciure, etc.) sont amenés par le transporteur 1, ainsi que les éventuels ajouts nutritifs par la ligne 2, dans un mélangeur-broyeur 3 où la granulométrie des résidus est maintenue dans une fourchette de 5 à 40 mm, généralement entre 6 et 12 mm. On incorpore également à ces matières solides, par le circuit 4, les boues ou résidus pâteux extraits du fond du digesteur (voir ci-après) de façon à conférer aux matières premières une humidité optimale - par exemple de 50 à 70 % selon les types de substrats - et à leur apporter déjà les concentrats de micro-organismes facilitant la dégradation. Quant aux déchets liquides - par exemple ici des eaux résiduaires brutes - ils sont tout d'abord amenés par la ligne 5, dans un séparateur solide-liquide 6, par exemple de type tamis à tambour filtrant, de façon à récupérer par la ligne 7 les résidus semi-solides que l'on associe aux déchets précités, le filtrat étant envoyé par la ligne 8 dans un bac de stockage 9. Ce bac peut avoir un compartiment 10 de nouvelle sédimentation d'où les produits solides sont évacués par le circuit 31 jusqu'à la ligne 7, alors que, dans la bâche 11 de réception du liquide, on procède avantageusement à une acidification pour faciliter la première phase d'acidogénèse de la fermentation anaérobie.

Pour l'étape opérationnelle de fermentation B, on dirige par la ligne 12 l'ensemble des déchets solides ou semi-solides sur le tas 13 de fermentation à l'air, alors que la masse liquide du récipient 11 est amenée par la pompe 14 et la canalisation 15 dans le digesteur clos 16. Le processus de dégradation aérobie des déchets solides ou assimilés s'effectue, selon l'invention, sur toute la périphérie du digesteur 16 et il peut bien entendu être accéléré par insufflation d'air et apport de compléments de microflore pour obtenir des rapports C/N et C/P les plus appropriés à la production de compost fertilisant, selon les types de matières premières mises en oeuvre. La chaleur dégagée par cette fermentation, dont la durée peut varier par exemple de trois à vingt jours environ, sert au chauffage naturel et permanent du digesteur 16.

Comme on peut le voir, sur les figures 1 à 4, le digesteur 16 est divisé en deux compartiments I et II séparés par un plafond 17 à poutres 18 et entre lesquels des communications sont établies par des tubes latéraux 19 et la canalisation centrale 20 au sein de laquelle est amenée, par la conduite 15, la masse liquide à fermenter, par exemple de bas en haut. Selon une caractéristique de l'invention, la pression du gaz généré dans ce bioréacteur 16 sert à l'agitation pulsatoire de la masse à traiter. En effet, pendant cette génération de gaz ($CH_4$ et $CO_2$) la pression qui existe dans le compartiment I provoque le déplacement du liquide d'un certain niveau dans le compartiment II par le(s) tuyau(x) 19. A un niveau donné, l'ouverture de la vanne 21 d'évacuation du gaz du compartiment I donne la possibilité au liquide accumulé dans le compartiment II de descendre rapidement par le siphon 20 vers le fond du fermenteur, ce qui produit un mélange et un brassage de la biomasse avec l'effluent liquide à traiter. Entre deux pulsations, la sédimentation du lit de boues en I et II se produit à contre-courant de l'effluent à traiter, alors que, dans les deux compartiments, a lieu la clarification. Cette dernière est facilitée par la mise en place dans ces compartiments, de sériesde plaques

0172292

verticales parallèles 23 dans le compartiment I, ces plaques reposant sur une grille 24, et des lamelles inclinées parallèles 23' dans le compartiment II.

Le niveau variable obtenu en permanence dans le digesteur empêche la formation d'une croûte dure; en cas d'apparition de celle-ci, elle est cassée et remise en solution grâce à l'action des poutres 18 fixées au plafond 17 du compartiment I. Les boues 22 accumulées au fond du radier sont, comme dit ci-dessus, renvoyées par la canalisation 4 au mélangeur - broyeur 3 pour apporter l'humidité nécessaire aux déchets concassés solides.

Dans la zone C ont lieu les évacuations de produits traités et générés, à savoir: la récupération du biogaz en 25, 25' avec dispositifs de commande et sécurité 26, soit vers un gazomètre, soit directement depuis celui-ci lorsqu'il est constitué par une cloche 27 au-dessus du digesteur 16 (figure 2); le soutirage en 28 de l'effluent traité, par exemple de l'eau épurée, si le résidu liquide amené en 5 est constitué par des eaux résiduaires; le soutirage en 4 des boues recyclées; et, enfin, l'évacuation en 29 du compost fermenté pour l'utilisation, par exemple, comme amendement agricole.

En pratique, le digesteur est en outre équipé des éléments classiques pour ce type d'appareillage et non représentés, dans un but de simplification, sur les figures, dont: hublots de surveillance, trous d'homme, échelle d'accès, systèmes de sécurité et contrôle de température, pression, etc. Afin d'obtenir un bon coefficient de transmission thermique, il peut être muni sur sa surface extérieure, sauf celle en contact avec le compost, de matériaux tels que: matelas de laine de roche, grillage de maintien et revêtement de protection en toile de jute et enduit bitumineux. Le radier est également et de préférence calorifugé.

0172292

En production industrielle, les ensembles de fermenteurs ou composteurs aérobie et de digesteurs anaérobie selon l'invention peuvent être agencés selon diverses dispositions, comme par exemple en série comme indiqué sur la figure 3 ou encore en parallèle selon l'illustration de la figure 4. Dans le premier cas, l'effluent liquide 28' issu du premier digesteur est réintroduit dans le conduit central 20 du second digesteur avant sa récupération finale en 28 alors que, dans le second cas, l'alimentation des digesteurs par les déchets liquides à traiter se fait indépendamment par 15 et 15'. Dans les deux cas, les productions de biogaz, selon 25 et 25', sont regroupées ainsi que les recyclages 4 des boues en excès et les alimentations 12 en compost.

Conformément à une réalisation intéressante, les ensembles précités sont conçus pour utiliser au maximum la chaleur dégagée par le processus aérobie et les transferts thermiques aux bioréacteurs clos. Une mise en place particulièrement intéressante est illustrée schématiquement sur la figure 5 où l'on voit que les digesteurs 16, 16', 16" etc. sont enfermés, selon une structure en "nid d'abeilles", entre les cloisons 30, 30', 30" etc, ici hexagonales et par exemple en béton, des fermenteurs à l'air libre où les déchets solides humidifiés 13 subissent le compostage.

Comme on le comprendra par la description qui précède, l'installation selon l'invention est conçue pour le traitement de tous types de matières résiduelles organiques (cellulosiques, protéiques, polysaccharidiques, lipidiques, etc.) tant sous forme solide que sous forme pâteuse et/ou liquide et l'on peut, bien entendu, sans sortir du cadre de l'invention, prévoir diverses variantes d'adaptation et des dimensionnements appropriés selon les types de substrats utilisés et les taux de productions des produits liquides et gazeux désirés.

0172292

## Revendications

1. Procédé de traitement simultané de déchets organiques solides ou semi-solides et de déchets organiques liquides en vue de la production simultanée de compost et de biogaz, caractérisé en ce que les déchets organiques liquides sont soumis à une séparation liquide-solide, en ce que la phase liquide provenant de cette séparation est soumise à une fermentation anaérobie dans au moins un digesteur fermé, en ce que la phase solide provenant de ladite séparation liquide-solide est mélangée auxdits déchets organiques solides ou semi-solides, en ce que le mélange résultant est soumis à une fermentation aérobie à la périphérie dudit digesteur et en contact avec celui-ci, et en ce que l'on évacue dudit digesteur respectivement des boues, du liquide clarifié et du gaz, tandis que l'on récupère à la périphérie dudit digesteur du compost provenant de la fermentation aérobie desdits déchets solides ou semi-solides.

2. Procédé selon la revendication 1, caractérisé en ce qu'une partie au moins des boues séparées dans ledit digesteur est mélangée auxdits déchets organiques, solides ou semi-solides préalablement à leur introduction dans le digesteur.

3. Procédé selon l'une des revendications 1 et 2, caractérisé en ce que l'on ajoute auxdits déchets organiques des microorganismes fixés sur des supports.

4. Procédé selon l'une des revendications 1 à 3, caractérisé en ce que ledit digesteur comporte deux compartiments superposés, l'un supérieur à basse pression et l'autre inférieur à haute pression, lesdits compartiments communiquant entre eux, d'une part, par au moins un conduit latéral, d'autre part, par un siphon central, des moyens étant prévus pour abaisser, lorsque le liquide y atteint un niveau prédéterminé, la pression du compartiment inférieur.

5. Installation pour le traitement simultané de déchets organiques solides ou semi-solides et de déchets organiques liquides en vue de la production simultanée de compost et de biogaz, caractérisée en ce qu'elle comprend: des moyens (6,9) pour séparer les déchets organiques liquides en une phase solide et une phase liquide; au moins un digesteur fermé (16); des moyens (14, 15) pour introduire ladite phase liquide dans ledit digesteur; des moyens (31, 3) pour mélanger ladite phase solide auxdits déchets solides ou semi-solides; des moyens (12) pour amener le mélange résultant à la périphérie dudit digesteur (16) en contact avec celui-ci; et des moyens (25, 28, 4) pour évacuer respectivement dudit digesteur, d'une part, le gaz qui s'y forme par fermentation anaérobie, d'autre part, les boues qui s'y déposent, enfin, la phase liquide clarifiée.

6. Installation selon la revendication 5, caractérisée en ce qu'elle comporte des moyens (4) pour mélanger une partie au moins des boues fermentées dans le digesteur auxdits déchets solides ou semi-solides préalablement à leur introduction dans ledit digesteur.

7. Installation selon l'une des revendications 5 et 6, caractérisée en ce que ledit digesteur (16) comporte deux compartiments (I et II) superposés, un compartiment supérieur (II), à basse pression, et un compartiment inférieur (I) à haute pression, lesdits compartiments communiquant entre eux, d'une part, par au moins un conduit latéral (19), et, d'autre part, par un siphon central (20), des moyens (21) étant prévus pour abaisser sur commande la pression du compartiment inférieur, lorsque le liquide y atteint un niveau prédéterminé.

8. Installation selon la revendication 7, caractérisée en ce que le compartiment inférieur (I) du digesteur (16) comprend des plaques verticales (23) parallèles, tandis que ledit compartiment supérieur (II) comporte des lamelles parallèles (23') inclinées.

- 13 -

9. Installation selon l'une des revendications 5 à 8, caractérisée en ce que lesdits compartiments inférieur (I) et supérieur (II) dudit digesteur (16) sont séparés par un plancher au-dessous duquel des poutres (18) font saillie dans ledit compartiment inférieur.

10. Installation selon l'une des revendications 5 à 9, caractérisée en ce que l'alimentation en phase liquide dudit digesteur (16) s'effectue par ledit siphon (20).

11. Installation selon l'une des revendications 5 à 10, caractérisée en ce qu'elle comprend une pluralité de cloisons (30, 30', 30") définissant une structure en nid d'abeilles et une pluralité de digesteurs (16, 16', 16"), chaque digesteur étant logé dans une cellule distincte de ladite structure.

FIG.1

0172292

1/3

FIG.2

FIG.5

0172292

FIG.3

FIG.4

Office européen
des brevets

**RAPPORT DE RECHERCHE EUROPEENNE**

0172292

Numéro de la demande

EP 84 40 1707

## DOCUMENTS CONSIDERES COMME PERTINENTS

| Catégorie | Citation du document avec indication. en cas de besoin. des parties pertinentes | Revendication concernée | CLASSEMENT DE LA DEMANDE (Int Cl 4) |
|---|---|---|---|
| Y | FR-A-2 376 827 (G.F. SHATTOCK)<br><br>* Page 12, revendications 1,2,5; page 7, lignes 13-38 * | 1,2,5, 6,8 | C 02 F 11/02<br>C 02 F 11/16<br>C 05 F 13/00<br>C 12 M 1/00 |
| | --- | | |
| Y | FR-A- 905 541 (J. TISSEYRE)<br><br>* Page 4, résumé; figure 1 * | 1,2,5, 6,8 | |
| | --- | | |
| A | FR-A-1 268 165 (L. MERLI)<br>* Page 1, lignes 1-9; résumé * | 3 | |
| | --- | | |
| A | FR-A- 818 680 (G. DUCELLIER et al.)<br>* Page 3, résumé * | 4,7,10 | |
| | --- | | DOMAINES TECHNIQUES RECHERCHES (Int. Cl.4) |
| A | WATER RESEARCH, vol. 11, 1977, pages 295-304, Pergamon Press, GB; E.S.K. CHIAN et al.: "Treatment of high strength acidic wastewater with a completely mixed anaerobic filter"<br>* Page 296, figure 1 * | 8 | C 02 F<br>C 12 M<br>C 05 F |
| | --- | | |
| A | DE-A-1 584 857 (ANLAGENBAU-GESELLSCHAFT)<br>* Page 5, alinéa 2; figure 1 * | 11 | |
| | --- | | |
| L | FR-A-2 541 669 (MULTIBIO) | | |
| | ----- | | |

Le present rapport de recherche a été établi pour toutes les revendications

| Lieu de la recherche | Date d'achèvement de la recherche | Examinateur |
|---|---|---|
| LA HAYE | 17-04-1985 | TEPLY J. |